(19) **Europäisches Patentamt / European Patent Office / Office européen des brevets**

(11) **EP 2 196 539 B1**

(12) **EUROPEAN PATENT SPECIFICATION**

(45) Date of publication and mention
of the grant of the patent:
**09.02.2022 Bulletin 2022/06**

(51) International Patent Classification (IPC):
**C12P 5/02** (2006.01)     **C12P 7/64** (2022.01)

(52) Cooperative Patent Classification (CPC):
**C12P 5/02; C12P 7/6463;** Y02E 50/30

(21) Application number: **08171701.9**

(22) Date of filing: **15.12.2008**

(54) **Process for the continuous biological production of lipids, hydrocarbons or mixtures thereof**

Verfahren zur kontinuierlichen biologischen Herstellung von Lipiden, Kohlenwasserstoffen oder Zusammensetzungen davon

Procédé pour production continue biologique des lipides, glucides ou leurs mélanges

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**16.06.2010 Bulletin 2010/24**

(73) Proprietor: **Delft Advanced Biofuels B.V. 2613 AX Delft (NL)**

(72) Inventors:
• **Van der Wielen, Lucas Antonius Maria 2665 AR Bleiswijk (NL)**
• **Heijnen, Johannes Joseph 5122 KK Rijen (NL)**

(74) Representative: **V.O. P.O. Box 87930 2508 DH Den Haag (NL)**

(56) References cited:
WO-A-03/050274     WO-A-2007/104551
WO-A-2007/139924     WO-A-2008/113041
US-A- 4 127 447

• **KALSCHEUER R ET AL: "Microdiesel: Escherichia coli engineered for fuel production" MICROBIOLOGY, SOCIETY FOR GENERAL MICROBIOLOGY, READING, GB, vol. 152, 1 January 2006 (2006-01-01), pages 2529-2536, XP007903430 ISSN: 1350-0872**
• **VEENSTRA J.N. ET AL: "Refinery wastewater management using multiple-angle oil water separators"[Online] 1998, pages 1-22, XP002529634 Mohr Separations Research, Inc. Retrieved from the Internet: URL:http://www.msrows.com/adobe/12Refinery wastewatermanagement.pdf> [retrieved on 2009-05-26]**
• **QIANG LI ET AL: "Perspectives of microbial oils for biodiesel production" APPLIED MICROBIOLOGY AND BIOTECHNOLOGY, SPRINGER, BERLIN, DE, vol. 80, no. 5, 9 August 2008 (2008-08-09), pages 749-756, XP019654113 ISSN: 1432-0614**
• **ARJAN S. HEERES ET AL: "Fermentation broth components influence droplet coalescence and hinder advanced biofuel recovery during fermentation", BIOTECHNOLOGY JOURNAL, vol. 10, no. 8, 1 August 2015 (2015-08-01), pages 1206-1215, XP055501183, DE ISSN: 1860-6768, DOI: 10.1002/biot.201400570**
• **HEERES ARJAN S ET AL: "Microbial advanced biofuels production: overcoming emulsification challenges for large-scale operation", TRENDS IN BIOTECHNOLOGY, vol. 32, no. 4, April 2014 (2014-04), pages 221-229, XP028837784, ISSN: 0167-7799, DOI: 10.1016/J.TIBTECH.2014.02.002**

**Description**

The invention is directed to a process for the continuous biological production of a product selected from the group of lipids, hydrocarbons, organic acids, polyalknoates and mixtures thereof by conversion of a suitable substrate using micro-organisms, in which process the said substrate is continuously, anaerobically fermented to produce the product and fermentation gas, in the presence of, optionally supported, micro-organisms.

Direct fermentative production of lipids, hydrocarbon biofuels (hereafter also indicated as BHC) instead of alcohols such as ethanol and butanol, has a tremendous potential benefit in terms of fuel purification costs (both capital and energy) as well as fuel properties (blending, corrosion, hygroscopy, etc). Both rely on the low water-miscibility characteristics of lipids or hydrocarbon(-like) molecules . Production of long chain lipids and fatty acids is well known in living organisms as common energy storage mechanism, and several new industries claim fast progress in commercial development of "product similar to conventional refined fuels" (Amyris) or "products similar to crude oil" (LS9). Although little detail is published, direct excretion of these products seems to be key in their development. These developments are still directed to the principles of the production of the BHC at laboratory scale and the selection or generation of the micro-organisms that may be used therein. In general, the fermentation is performed in stirred fermentors and the product is separated from the microbial cells or broth by conventional solid-liquid separation centrifuges.

In WO2007/139924, Amyris further describes a production system for making bio-organic compounds, e.g. for a biofuel. The system may include a vessel, a first phase comprising an aqueous medium including host cells capable of producing a bio- organic compound, where the bio-organic compound comprises a second phase in contact with the aqueous medium.

US 4,127,447 relates to a process for carrying out a biologically catalyzed reaction comprising the steps of: introducing an influent solution containing reactants into an upflow packed bed column packed with a support having attached thereto anaerobic microorganisms for catalyzing the reaction of said reactants; introducing with said influent a membrane disruptive detergent to lyse dead microorganisms from said support and to make available the essential nutrient of said dead microorganisms to the remaining live microorganisms. The process is in particular for use in the treatment of industrial organic wastes and domestic sewage. WO2003/050274 relates to a method for producing a crystalline and/or amorphous metabolite suspension from a fermentation broth comprising treating the fermentation broth with one or more coagulants and/or one or more flocculants; and separating the biomass of the fermentation broth from the coagulated and/or flocculated fermentation broth by use of a separation equipment, whereby a crystalline and/or amorphous metabolite suspension is obtained.

WO2007/104551 relates to a process for the purification of waste water, in particular for the continuous purification of waste water in papermaking, where waste water to be purified is treated with anaerobic microorganisms in order to degrade contaminants present in the waste water. The process further comprises subjecting waste water to a pressure-release flotation step before being supplied to the anaerobic reactor or after being discharged from the anaerobic reactor, in order to delime the water to at least some extent.

For reasons of yield and costs, a biological process without external electron acceptor (such as oxygen) is desired. The anaerobic fermentation also leads (stoichiometrically) to substantial carbon dioxide (or other fermentation gasses like methane and hydrogen) production rates, sufficient for turbulent mixing and dispersion in the reactor without requiring a separate mixing device.

This results in a four phase (S-G-L-L) mixture: microbial cells, fermentation gas, aqueous medium and (liquid) BHC. BHC may be present as small (<10 $\mu$m), medium (10-100 $\mu$m), large (>100 $\mu$m) droplets or a combination thereof.

It is an object of the present invention to provide a commercially and technically feasible process at a scale that can compete with crude oil recovery, while using renewable sources, in general bio-based feedstocks such as sugars, or in more general terms, carbohydrates, starch, hydrolysates of cellulose and the like. Other suitable feedstocks may be derived from glycerol, low cost biofuels (methanol, ethanol and the like), agro/forestry residuals, hydrolysates of agro/forestry residuals and gasified forms thereof (for example syngas).

Fermentation gas is commonly composed of mostly carbon dioxide and some water vapour, but could contain substantial amounts of methane. The fermentation medium can also contain other, oxidized, products next to BHC such as organic acids and polymers thereof (for example polyalkanoates). Such a presence does not limit the invention.

It is also an object of the invention to provide an integrated bioprocess for advanced biofuels. The present invention aims at providing a low cost, integrated bioreactor with several technology options for the direct production of a BHC-stream from renewables.

These and other objects are obtained by the process for the continuous biological production of a product selected from the group of lipids, hydrocarbons, organic acids, polyalkanoates and mixtures thereof by conversion of a suitable substrate using micro-organisms, in which process the said substrate is continuously, anaerobically fermented to produce the product and fermentation gas, in the presence of, optionally supported, micro-organisms in an aqueous medium in a column type reactor, in which reactor at least part of the aqueous medium flows in upward direction, and recovering the product by separating the fermentation gas, the micro-organisms in the aqueous medium and the product from each other under conditions that coalescence of the product is promoted,
wherein the fermentation gas is at least partly separated from the aqueous medium prior to separating the product from the aqueous phase wherein SLL (i.e. solid-liquid-liquid) separation techniques are used for promoting the coalescence of the product droplets in order to enhance separation, said SLL separation techniques including gravity settling, flotation or a combination thereof, wherein the separation of the product is done using a tilted plate separator or wherein the product is separated from the aqueous medium by flotation using fermentation gas dissolved in the aqueous medium, wherein fermentation gas dioxide is generated for flotation by a pressure decrease of the aqueous medium..

The present invention is based on the combined and integrated use of a reactor, the specific properties of the main products (fermentation gas and BHC) and various downstream processing unit operations in an inventive way, for a specific process configuration.

An important aspect of the process is the anaerobic fermentation of the substrate, using suitable micro-organism, which may optionally be supported. Suitable micro-organisms are known in the art, for example in WO 2008/113041 (LS9), Kalscheuer R, Stölting T and Steinbüchel A. 2006. Microdiesel: Escherichia coli engineered for fuel production. Microbiology 152: 2529-2536 or in Li Q, Du W and Liu D. 2008. Perspectives of microbial oils for biodiesel production. Applied Microbiology and Biotechnology 80: 749-756.

The micro-organisms can be freely suspended, immobilized, forming granules, forming flocs and the like. In this way the cell concentration in the reactor can be optimized and less surplus cell mass is produced. Additionally, separation of the cell mass may be enhanced. The process is basically in up-flow with recirculation over the column reactor. This may either be an internal recirculation, for example using a draft tube, or an external recirculation.

In the process a large amount of fermentation gas is generated, which generates turbulent mixing and dispersion in the reactor and provides a useful gas lift function in the reactor. In specific embodiments the fermentation gas also has a useful function in the separation of the BHC from the reaction broth.

Liquid withdrawal can be done from the top or from the bottom of the reactor. In specific embodiments of the invention liquid withdrawal from the bottom of the reactor is preferred since it enhances the subsequent separation steps by making use of the pressure head of the reactor. The reactor pressure can be further controlled by regulating the fermentation gas flow leaving the reactor.

In combination with the above aspect of the carbon dioxide generation and use, there is the aspect of the separation of the various phases in the reaction mixture or broth, namely gas (fermentation gas), two liquid phases (aqueous medium and the apolar lipids, hydrocarbon, organic acid, or polyalkanoate) and an optional solid phase (optionally supported micro-organisms). In order to recover the product of the process of the invention, it is important that the separation steps include one wherein the conditions are such that the coalescence of the BHC is promoted or stimulated.

The droplets of the product that is produced in the process of the invention may have varying sizes, ranging from small (<10 $\mu$m), medium (10-100 $\mu$m) or large (>100 $\mu$m).

The BHC is generally present in emulsified form, which may be stabilized by the presence of micro-organisms and other biological particles and molecules. This requires additional measures to make sure that the BHC coalesce and may be recovered as a separate liquid phase. In some instances, a (generally small) part of the product may be gaseous, which would then require a separate step to recover them form the carbon dioxide based gas phase.

Separation techniques for separating oil from water are known in the art. E.g. Veenstra et al. (Refinery wastewater management using multiple angle oil water separators (1998), p1-22, Mohr Separations Research) is directed to the field of waste water management of fossil oil production and fossil oil refinery.

The SSL separation includes the use of an SSL separation technique selected from gravity settling, flotation, combinations thereof, wherein the separation of the product is done using a tilted plate separator or wherein the product is separated from the aqueous medium by flotation using fermentation gas dissolved in the aqueous medium.

Several separation techniques may be used for separating the product and/or promoting the coalescence of the product (solid-liquid-liquid separation, in short SLL separation). The selection of the separation technique depends on the properties of the mixture and the coalescence properties of the product droplets. The said SLL separation techniques are used for separating the BHC and/or for promoting the coalescence of the BHC droplets in order to enhance separation. Further examples of suitable SLL separation techniques that may additionally be used include centrifugation, use of hydrocyclones, filtration, membrane filtration or a combination thereof.

Preferred steps for this include the use of flotation of the BHC using dissolved carbon dioxide and other components in the fermentation gas in combination with a ventury type device (or other pressure reduction devices) or the use of hydrocyclones. These techniques can be used when small droplets of the product are present in the mixture. In specific embodiments these steps can also be combined with each other, or they may be combined with other separation systems, such as a tilted plate separator. A tilted plate separator can be used when large droplets of the product are present in the mixture. It is, for example, possible to separate at least part of the fermentation gas, the solids and the product from the aqueous phase in such a separator.

As has been indicated, the process requires a recirculation over the reactor. This may be done by an internal recirculation of part of the reaction broth, using an internal draft tube, possibly after separating carbon dioxide and part of the BHC produced. It is also possible to recirculate the solids, with part of the aqueous medium, but with relatively low amounts of BHC and carbon dioxide, through an external recirculation, after the separation of the major amount of the carbon dioxide and BHC from it.

Although fermentation generally has a relatively low volumetric production rate, the process of the present invention overcomes this disadvantage by the possibility to use large amounts of cell mass and the direct separation of the product from the reaction broth. In addition the process of the invention has the advantage of a limited number of unit operations and only those that do not have moving parts, instead of centrifuges. Further, the nature of the materials involved allows the use of relatively cheap construction materials, compared to conventional fermentors, namely no need to use steel fermentors, but concrete or plastics suffice. Also there is little possibility of erosion, which also makes the use of lower cost materials possible. These advantages result in considerable reductions in investment and operational costs compared to conventional technologies.

The process is based on the bioconversion of renewable substrates, mainly carbohydrates, but also other materials, as defined above. These substrates are generally introduced into the reactor as aqueous solution, either directly into the reactor or into the recycle flow, if present.

The reactor may be operated at ambient pressure, but in case the fermentation gas flotation method, discussed above, is used, it is preferred that the reactor is operated at a slightly higher pressure, such as between 0.11 MPa and 2.0 MPa (abs) (1.1 and 20 bar (abs)), thereby enabling the flotation by a simple pressure reduction after the reactor. It is also possible to operate the reactor under ambient pressure and to withdraw the liquid from the bottom. Because of the pressure head of the reactor contents, up to 10 meters of water column or more, there is enough carbon dioxide dissolved in the liquid for the required flotation by carbon dioxide. In the alternative, it is also possible to use this pressure to drive the hydrocyclones. The reactor pressure can be further controlled by regulating the carbon dioxide flow leaving the reactor.

In the figures, three different flow sheets of embodiments of the invention are provided. In Figure 1 a general flow scheme based on flotation is given, in Figure 2 a flow scheme using hydrocyclones is given and in Figure 3 a flow scheme combining hydrocyclones and flotation is given.

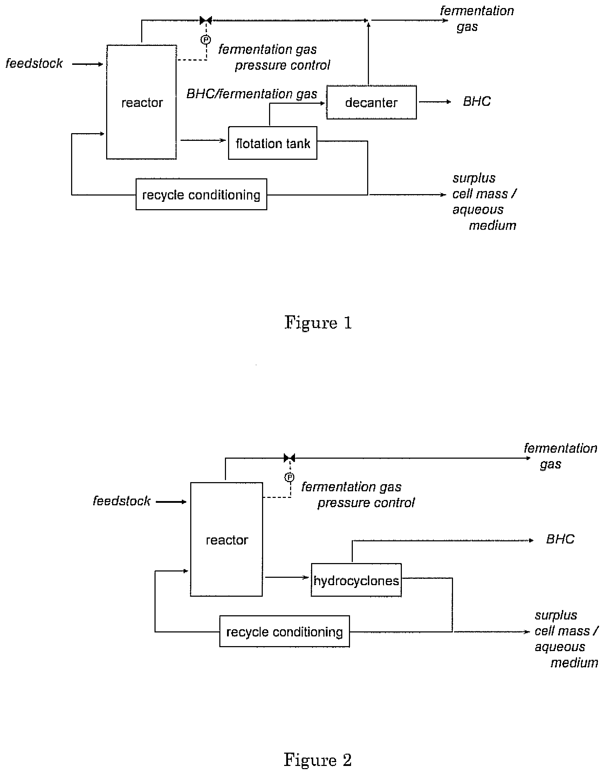

In Figure 1, the liquid effluent port is placed at the bottom of the reactor. Due to the static pressure at the bottom of the reactor, a substantial amount of fermentation gas is absorbed in the broth. Eventually, the amount of fermentation gas absorbed in the broth can be increased by increasing the pressure in the reactor, for example by controlling the flow of fermentation gas leaving the reactor. The BHC-broth suspension leaves the reactor with a high fermentation gas pressure. Releasing the pressure in the flotation tank results in small bubbles that selectively accumulate small droplets of BHC. BHC is then recovered from the bubbles in a decanter, while the broth (and eventually remaining BHC) can be (partly) recycled to the reactor.

In Figure 2, the liquid effluent is placed at the bottom of the reactor. The hydrostatic pressure of the reactor may be sufficient to drive the hydrocyclones at no further operational (energy) costs. Eventually, the pressure in the reactor can be increased by, for example, controlling the flow of fermentation gas leaving the reactor. The centrifugal field in the hydrocyclones may be sufficient to ensure the BHC separation with the overflow, while the broth (and eventually remaining BHC) can be (partly) recycled to the reactor.

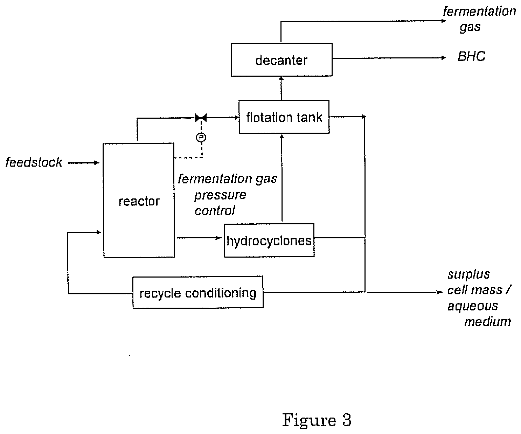

The centrifugal field in the hydrocyclones may concentrate small BHC droplets towards the center of the hydrocyclone and promote coalescence. Improved BHC recovery may thus be achieved by combining the use of hydrocyclones and flotation as shown in Figure 3.

The invention is now elucidated on the basis of examples.

EXAMPLES

**1. Anaerobic production of hydrocarbon from glucose**

This example illustrates typical flows over the reactor and their impact on the reactor size.

For dodecane ($C_{12}H_{26}$) as an example product and glucose ($C_6H_{12}O_6$) as an example substrate, the catabolic reaction for product formation is:

$$3\tfrac{1}{12}\, C_6H_{12}O_6 \rightarrow 1\, C_{12}H_{26} + 6.5\, CO_2 + 5.5\, H_2O \qquad (1)$$

This results in a maximum yield on glucose of

$$Y_{ps} = \frac{3\,\frac{1}{12}\,mol\ glucose}{mol\ product} = 3.26\ kg\ glucose\,/\,kg\ product \qquad (2)$$

In this example, a plant producing 30 million gallon hydrocarbon per annum (120 000 $m^3$/year or 85 kton/year) is considered. Assuming an 8000 hour annual operation this results in a production rate of 11 ton/hour (59 kmol of product per hour).

From Equation (1) it can be seen that this leads to a substantial carbon dioxide ($CO_2$) production rate (388 284 mol $CO_2$/hour) which is more than sufficient for turbulent mixing and dispersion in the reactor, and therefore, no separate mixing device is required.

For reactor-sizing purposes, a micro-organism growing at a rate $\mu$ = 0.06 1/hour is considered. Typical substrate consumption and cell mass production rates are $R_S$ = 198 kmol/hour and $R_X$ = 87 kmol/hour respectively. With these values, the required substrate feed can be calculated. For a glucose feed concentration of 180 g/kg this results in a feed flow $F^{in}$ = 198 ton/hour. The water outflow is the sum of the water inflow with the feed and the stoichiometric reaction water, resulting in an outflow $F_w^{out} = 168.2$ ton/hour. Furthermore, the cell mass concentration can be calculated from

$$C_X = \frac{R_X}{F_w^{out}} = 516\ C\text{-}mol\ cells\,/\,m^3 \qquad (3)$$

In this example, the specific production rate by the micro-organism is assumed to be $q_P$ = 0.041 mol product/mol cells/hour. In order to produce 59 kmol product/hour, $1.45 \times 10^6$ C-mol cells are required. Based on an average cell molecular weight composition of 24.6 g/C-mol, this results in about 36 ton cell mass in the reactor. Assuming a well-mixed reactor and from the result of Equation (3), 2 810 $m^3$ water are present in the reactor. The volume of product in the reactor is 252 $m^3$. Assuming a $CO_2$ gas hold-up of 10% of the liquid suspension, the result is a total minimum reactor volume of 3 343 $m^3$. In order to ensure good mixing, the reactor is designed for a superficial gas velocity of 70 m/hour, resulting in a cross-sectional area of 143 $m^2$ (or a diameter of 6.7 m) and a height of 23 m. Clearly, these values are strongly dependant on the type of product, the required production rate, the micro-organism used and the growth rate of the micro-organism, among others.

## 2. Product separation by means of a tilted plate settler

This example illustrates the effect of the product droplet size on the selection of the separation technique.

Considering the reactor of Example 1, the reactor is operated at atmospheric conditions. $CO_2$ is first separated, after which the product-broth mixture enters a tilted plate settler. The settler can be mounted on top of the reactor or external. The product droplet rise velocity is a strong function of the droplet diameter and can be calculated with the Stokes equation:

$$u = \frac{1}{18} \cdot \frac{g \cdot \Delta\rho \cdot d^2}{\eta} \qquad (4)$$

For the process described in Example 1, the density difference $\Delta\rho$ is 300 kg/$m^3$ and viscosity $\eta$= 10 mPa s. In order to compare different separation techniques, the sigma factor $\Sigma$ is commonly used and corresponds to the equivalent surface area of a settling tank with the same theoretical performance. Table 1 shows the droplet rise velocity u and the required sigma factor for different droplet sizes.

Table 1. Sigma factor as a function of droplet size.

| $d$ ($\mu$m) | u (m/hr) | $\Sigma$ / $m^2$ |
|---|---|---|
| 0,1 | 0,0000006 | 280 833 333 |
| 1 | 0,00006 | 2 808 333 |
| 10 | 0,006 | 28 083 |
| 100 | 0,6 | 281 |
| 1000 | 60 | 3 |

For comparison purposes, the sigma value of disc stack centrifuges is roughly limited to 100 000 m$^2$ and for tilted plate settlers to 1 000 m$^2$. It is clear that this separation method can only be used when sufficiently large product droplets are present in the mixture.

### 3. Effect of a micro-organism on droplet size and mixture stability

This example illustrates the range of droplet sizes that can be obtained and the coalescence behavior of the mixture.

Mixtures of water, dry baker's yeast and decane ($C_{10}H_{22}$) were prepared at ambient temperature in a 2L vessel equipped with a four bladed Rushton turbine operating at 900 rpm. After 3.5 hour the mixture was put in a 1L graduated cylinder in order to observe the separation behavior. Table 3 summarizes the mixtures evaluated.

Table 3. Mixture composition.

| Mixture | Water (mL) | Dry yeast (g) | Decane (mL) |
|---------|-----------|---------------|-------------|
| A | 900 | 10 | 93 |
| B | 990 | 10 | - |
| C | 910 | - | 90 |
| D | 900 | 10 | 90 |

Mixture A. After 12 minutes settling, two phases had formed: an upper layer with large, coalesced decane droplets and yeast cells and a layer (about 800 mL) containing mostly yeast cells and decane droplets in varying sizes.

Mixture B. In this mixture, no decane was added in order to evaluate the settling behavior of the cells in water without influence of the product. After 20 hours there was no clear water layer but 3 fractions were clearly differentiated: a top layer with low cell concentration, a middle layer with high cell concentration and a bottom layer (approx. 0.68 cm) with settled cells. Using Stokes equation a particle diameter of 2 μm was calculated, which is a typical diameter for bakers' yeast cells.

Mixture C. In this mixture, no yeast cells were added in order to evaluate the rising behavior of the decane droplets without influence of the micro-organism. The mixture was unstable as expected and about 84 mL of decane was clearly separated within 1 minute.

Mixture D. This mixture is similar to Mixture A. After 21 hours of settling most of the decane was present in the top layer. Two 40 mL samples were taken from this layer and centrifuged for 30 minutes at 4700 rpm. The fractions obtained are summarized in Table 4.

Table 4. Fractions obtained after centrifugation of two samples from the top layer (after 21 hours settling) of Mixture D.

| Sample | Total volume | yeast cells | clear water | mixture | clear decane |
|--------|-------------|-------------|-------------|---------|--------------|
| 1 | 40 mL | approx. 1 mL | 7 mL | 8 mL | 24 mL |
| 2 | 40 mL | approx. 0.5 mL | 3 mL | 4 mL | 32.5 mL |

These experiments indicate a wide drop range from small (< 10 μm) to large (> 100 μm) drops, with many drops in the small to medium range. (Near) complete recovery requires classically centrifugation, which is too costly given the fuel-use of the BHC product. The present invention uses dissolved fermentation gas, which can flotate small drops or hydrocyclones that coalesce small drops to larger ones and separate those.

### 4. Product separation by means of flotation through dissolved fermentation gas

This example illustrates the effect of the product droplet size on the selection of the separation technique.

The bottom zone of the reactor described in Example 1 has a static pressure of 0.2 MPa (2 bars), where a substantial amount of $CO_2$ is absorbed in the broth. With a configuration as shown in Figure 1, small product droplets can be separated from the mixture. The flotation efficiency strongly depends on the droplet-bubble diameter ratio, their velocities and abundance. Following the same approach as in Example 2 the sigma factor is shown in Table 2.

Table 2. Sigma factor as a function of droplet size.

| $d$ ($\mu$m) | u (m/hr) | $\Sigma$ / m$^2$ |
|---|---|---|
| 10 | 0,02 | 8425 |
| 100 | 2 | 84 |
| 1000 | 200 | 1 |

It is clear that this separation method can be used when small product droplets are present in the mixture.

**Claims**

1. Process for the continuous biological production of a product selected from the group of lipids, hydrocarbons, organic acids, polyalkanoates and mixtures thereof, wherein said products are able to form droplets in the aqueous fermentation medium,

   by conversion of a suitable substrate using micro-organisms,
   in which process the said substrate is continuously, anaerobically fermented to produce the product and fermentation gas, in the presence of, optionally supported, micro-organisms in an aqueous medium in a column type reactor, in which reactor at least part of the aqueous medium flows in upward direction, and recovering the product by separating the fermentation gas, the micro-organisms in the aqueous medium and the product from each other under conditions that coalescence of the product is promoted,
   wherein the fermentation gas is at least partly separated from the aqueous medium prior to separating the product from the aqueous phase wherein SLL (*i.e.* solid-liquid-liquid) separation techniques are used for promoting the coalescence of the product droplets in order to enhance separation, said SLL separation techniques including gravity settling, flotation or a combination thereof, wherein the separation of the product is done using a tilted plate separator or wherein the product is separated from the aqueous medium by flotation using fermentation gas dissolved in the aqueous medium, wherein fermentation gas dioxide is generated for flotation by a pressure decrease of the aqueous medium.

2. Process according to claim 1, wherein the micro-organisms can be freely suspended, immobilized, forming granules, forming flocs and the like.

3. Process according to claim 1 or 2, wherein the substrate is selected from sugars, starch, glycerol, methanol, ethanol, agro/forestry residuals, hydrolysates of agro/forestry residuals and gasified forms thereof (for example syngas).

4. Process according to claim 1-3, wherein carbon dioxide is first separated from the medium in the upper of the column following which the product is separated from the aqueous medium containing micro-organisms, and wherein the aqueous medium containing micro-organisms is at least partly recirculated to the column.

5. Process according to claim 1-4, wherein the separation is done using a tilted plate separator.

6. Process according to claim 1-4, wherein the product is separated from the aqueous medium by flotation using fermentation gas dissolved in the aqueous medium, wherein fermentation gas dioxide is generated for flotation by a pressure decrease of the aqueous medium.

7. Process according to claim 6, wherein the reactor is operated under a pressure higher than 1 bar abs, preferably by regulating the outflow of fermentation gas from the reactor.

8. Process according to claim 1-7, wherein the reactor is an upflow reactor, a column reactor with internal or external recirculation, for example using a draft tube in the reactor.

9. Process according to claim 1-8, wherein the method is for the continuous biological production of hydrocarbons.

10. Process according to claim 1-9, wherein the method is for the continuous biological production of lipids.

**11.** Process according to claim 1-10, wherein a large amount of fermentation gas is generated, which generates turbulent mixing and dispersion in the reactor and provides a useful gas lift function in the reactor.

**Patentansprüche**

**1.** Verfahren zur kontinuierlichen biologischen Herstellung eines Produkts ausgewählt aus der Gruppe von Lipiden, Kohlenwasserstoffen, organischen Säuren, Polyalkanoaten und Mischungen davon, wobei die Produkte in der Lage sind, Tröpfchen in dem wässrigen Fermentationsmedium zu bilden, durch Umwandlung eines geeigneten Substrats unter Verwendung von Mikroorganismen, wobei im Verfahren das Substrat kontinuierlich anaerob fermentiert wird, um das Produkt und das Fermentationsgas zu erzeugen, in der Gegenwart von, optional unterstützten, Mikroorganismen in einem wässrigen Medium in einem Kolonnenreaktor, wobei im Reaktor wenigstens ein Teil des wässrigen Mediums nach oben strömt, und Gewinnen des Produkts durch Trennen des Fermentationsgases, der Mikroorganismen in dem wässrigen Medium und des Produkts voneinander unter Bedingungen, so dass Koaleszenz des Produkts gefördert wird,
wobei das Fermentationsgas wenigstens teilweise von dem wässrigen Medium getrennt wird, bevor das Produkt von der wässrigen Phase getrennt wird, wobei SLL (*d.h.* fest-flüssig-flüssig, ("solid-liquid-liquid")) -Trenntechniken zum Fördern der Koaleszenz der Produkttröpfchen verwendet werden, um die Trennung zu verbessern, die SLL-Trenntechniken einschließend Schwerkraftabscheidung, Flotation oder eine Kombination davon, wobei die Trennung des Produkts unter Verwendung eines Schrägplattenseparators erfolgt oder wobei das Produkt von dem wässrigen Medium durch Flotation unter Verwendung von in dem wässrigen Medium gelöstem Fermentationsgas getrennt wird, wobei Fermentationsgasdioxid zur Flotation durch eine Druckabsenkung des wässrigen Mediums erzeugt wird.

**2.** Verfahren nach Anspruch 1, wobei die Mikroorganismen frei suspendiert, immobilisiert, granulatbildend, flockenbildend und dergleichen sein können.

**3.** Verfahren nach Anspruch 1 oder 2, wobei das Substrat ausgewählt ist aus Zuckern, Stärke, Glycerin, Methanol, Ethanol, Land-/Forstwirtschaftsrückständen, Hydrolysaten von Land-/Forstwirtschaftsrückständen und vergasten Formen davon (zum Beispiel Syngas).

**4.** Verfahren nach Anspruch 1 - 3, wobei zunächst Kohlendioxid aus dem Medium in dem oberen Teil der Kolonne getrennt wird, wonach das Produkt von dem wässrigen Medium, enthaltend Mikroorganismen, getrennt wird, und wobei das wässrige Medium, enthaltend Mikroorganismen, wenigstens teilweise in die Kolonne zurückgeführt wird.

**5.** Verfahren nach Anspruch 1 - 4, wobei die Trennung unter Verwendung eines Schrägplattenseparators erfolgt.

**6.** Verfahren nach Anspruch 1 - 4, wobei das Produkt von dem wässrigen Medium durch Flotation unter Verwendung von in dem wässrigen Medium gelöstem Fermentationsgas getrennt wird, wobei Fermentationsgasdioxid für die Flotation durch eine Druckabsenkung des wässrigen Mediums erzeugt wird.

**7.** Verfahren nach Anspruch 6, wobei der Reaktor unter einem Druck von mehr als 1 bar abs. betrieben wird, vorzugsweise durch Regeln des Abflusses von Fermentationsgas aus dem Reaktor.

**8.** Verfahren nach Anspruch 1 - 7, wobei der Reaktor ein Aufströmreaktor, ein Kolonnenreaktor mit interner oder externer Rezirkulation, zum Beispiel unter Verwendung eines Saugrohrs in dem Reaktor, ist.

**9.** Verfahren nach Anspruch 1 - 8, wobei das Verfahren für die kontinuierliche biologische Herstellung von Kohlenwasserstoffen ist.

**10.** Verfahren nach Anspruch 1 - 9, wobei das Verfahren für die kontinuierliche biologische Herstellung von Lipiden ist.

**11.** Verfahren nach Anspruch 1 - 10, wobei eine große Menge an Fermentationsgas erzeugt wird, das eine turbulente Mischung und Dispersion in dem Reaktor erzeugt und eine nützliche Gasliftfunktion in dem Reaktor bereitstellt.

**EP 2 196 539 B1**

**Revendications**

1. Procédé pour la production biologique continue d'un produit choisi dans le groupe de lipides, hydrocarbures, acides organiques, polyalcanoates et mélanges de ceux-ci, dans lequel lesdits produits sont aptes à former des gouttelettes dans le milieu de fermentation aqueux, par conversion d'un substrat approprié en utilisant des micro-organismes,

dans lequel procédé ledit substrat est fermenté de manière continue, anaérobie pour produire le produit et du gaz de fermentation, en présence de, micro-organismes, éventuellement sur support, dans un milieu aqueux dans un réacteur de type colonne, dans lequel réacteur au moins une partie du milieu aqueux s'écoule dans une direction vers le haut, et récupération du produit en séparant le gaz de fermentation, les micro-organismes dans le milieu aqueux et le produit les uns des autres dans des conditions dans lesquelles la coalescence du produit est promue,

dans lequel le gaz de fermentation est au moins partiellement séparé du milieu aqueux avant la séparation du produit de la phase aqueuse dans lequel des techniques de séparation SLL (c'est-à-dire solide-liquide-liquide) sont utilisées pour promouvoir la coalescence des gouttelettes de produit afin de promouvoir la séparation, lesdites techniques de séparation SLL incluant une décantation par gravité, flottation ou combinaison de celles-ci, dans lequel la séparation du produit est réalisée en utilisant un séparateur à plaque inclinée ou dans lequel le produit est séparé du milieu aqueux par flottation en utilisant du gaz de fermentation dissous dans le milieu aqueux, dans lequel du dioxyde de gaz de fermentation est produit par flottation par une diminution de pression du milieu aqueux.

2. Procédé selon la revendication 1, dans lequel les micro-organismes peuvent être mis en suspension, immobilisés librement, formant des granulés, formant des flocons et semblables.

3. Procédé selon la revendication 1 ou 2, dans lequel le substrat est choisi parmi des sucres, de l'amidon, du glycérol, du méthanol, de l'éthanol, des résidus de l'agroforesterie, des hydrolysats de résidus de l'agroforesterie et des formes gazéifiées de ceux-ci (par exemple syngas).

4. Procédé selon les revendications 1-3, dans lequel du dioxyde de carbone est dans un premier temps séparé du milieu dans le haut de la colonne après quoi le produit est séparé du milieu aqueux contenant des micro-organismes, et dans lequel le milieu aqueux contenant des micro-organismes est au moins partiellement remis en circulation dans la colonne.

5. Procédé selon les revendications 1-4, dans lequel la séparation est réalisée en utilisant un séparateur à plaque inclinée.

6. Procédé selon les revendications 1-4, dans lequel le produit est séparé du milieu aqueux par flottation en utilisant du gaz de fermentation dissous dans le milieu aqueux, dans lequel du dioxyde de gaz de fermentation est produit par flottation par une diminution de pression du milieu aqueux.

7. Procédé selon la revendication 6, dans lequel le réacteur est mis en fonctionnement sous une pression supérieure à 1 bar abs, de préférence par régulation de l'écoulement de sortie du gaz de fermentation du réacteur.

8. Procédé selon les revendications 1-7, dans lequel le réacteur est un réacteur à écoulement vers le haut, un réacteur en colonne avec une recirculation interne ou externe, par exemple en utilisant un tube d'aspiration dans le réacteur.

9. Procédé selon les revendications 1-8, dans lequel le procédé est destiné à la production biologique continue d'hydrocarbures.

10. Procédé selon les revendications 1-9, dans lequel le procédé est destiné à la production biologique continue de lipides.

11. Procédé selon les revendications 1-10, dans lequel une grande quantité de gaz de fermentation est produite, laquelle produit un mélange turbulent et une dispersion dans le réacteur et fournit une fonction d'élévation utile de gaz dans le réacteur.

9

Figure 1

Figure 2

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007139924 A
- US 4127447 A
- WO 2003050274 A
- WO 2007104551 A
- WO 2008113041 A

**Non-patent literature cited in the description**

- **KALSCHEUER R ; STÖLTING T ; STEINBÜCHEL A.** Microdiesel: Escherichia coli engineered for fuel production. *Microbiology,* 2006, vol. 152, 2529-2536
- **LI Q ; DU W ; LIU D.** Perspectives of microbial oils for biodiesel production. *Applied Microbiology and Biotechnology,* 2008, vol. 80, 749-756
- **VEENSTRA et al.** Refinery wastewater management using multiple angle oil water separators. *Mohr Separations Research,* 1998, 1-22